# EUROPEAN PATENT APPLICATION

(11) **EP 3 295 799 A1**
(43) Date of publication of application: **21.03.2018**
(21) Application number: 17760021.0
(22) Date of filing: 28.02.2017
(51) Int. Cl.: A01N 63/00, A01C 1/08, A01N 63/02, A01P 3/00, A61K 35/742, A61P 31/10, C12N 1/20, C12N 1/14

(54) **ANTIFUNGAL AGENT, PESTICIDE, METHOD FOR CONTROLLING PLANT DISEASE BY MEANS OF MICROORGANISM, AND NOVEL BACILLUS SUBTILIS**

(30) Priority: 04.03.2016 JP 2016042659
(71) Applicant: Organic Tech Farm Corporation, Kyoto-shi, Kyoto 612-8374 (JP)
(72) Inventor: HARA, Tomijiro, Kyoto-shi Kyoto 6128374 (JP); TAKATSUKA, Yumiko, Kyoto-shi Kyoto 612-8734 (JP); SUGIMURA, Yuya, Yonezawa-shi, Yamagata 9920062 (JP); YOKOTA, Kenji, Tokyo 156-8502 (JP)
(74) Representative: J A Kemp
(86) International application number: PCT/JP2017/007903
(87) International publication number: WO 2017/150556

(57) **Abstract**

One aspect of the present invention is an antimicrobial agent including at least one of *Bacillus subtilis* and a culture of *Bacillus subtilis*, the *Bacillus subtilis* showing a peak at at least one mass-to-charge ratio (m/z) selected from the group consisting of a range of 3045.5±0.58 (m/z), a range of 6942.5±0.58 (m/z), and a range of 9140.5±0.58 (m/z) in mass analysis by means of matrix-assisted laser desorption ionization time-of-flight mass spectrometry (MALDI-TOF MS).

## Description

### Technical Field

### Cross Reference to Related Applications

The present application claims priority based on Japanese Patent Application No. 2016-042659 filed on March 4, 2016 (the entire disclosure is incorporated herein by reference).

The present invention relates to an antimicrobial agent, a pesticide, a method for preventing a plant disease caused by microorganisms, and a novel *Bacillus subtilis.*

### Background Art

Crop diseases caused by microorganisms are a problem in cultivating crops. For example, a method of sprinkling other microorganisms and preventing the diseases by means of antimicrobial substances produced by the microorganisms has been attempted in order to deal with the diseases. However, as described in Patent Document 1, for example, antimicrobial substances that exhibit antimicrobial activity against fungi and the like that infect rice plants are not known.

### Citation List

### Patent Document

Patent Document 1: JP 2003-199558A

### Summary of Invention

### Technical Problem

Therefore, it is an object of the present invention to provide a novel antimicrobial agent that exhibits antimicrobial activity against fungi and the like that infect rice plants, for example, a pesticide, a method for preventing a plant disease caused by microorganisms, and a novel *Bacillus subtilis.*

### Solution to Problem

As a result of having conducted diligent research to achieve the above-mentioned object, the present invention as follows was achieved.

An antimicrobial agent of the present invention includes at least one of *Bacillus subtilis* and a culture of *Bacillus subtilis*, wherein the *Bacillus subtilis* is a microorganism showing a peak at at least one mass-to-charge ratio (m/z) selected from the group consisting of a range of 3045.5±0.58 (m/z), a range of 6942.5±0.58 (m/z), and a range of 9140.5±0.58 (m/z) in mass analysis by means of matrix-assisted laser desorption ionization time-of-flight mass spectrometry (MALDI-TOF MS).

A pesticide of the present invention includes the antimicrobial agent of the present invention.

A method for preventing a plant disease caused by microorganisms of the present invention (also referred to as "prevention method" hereinafter) includes a contacting step of bringing the antimicrobial agent of the present invention into contact with a plant.

The novel *Bacillus subtilis* of the present invention (also referred to as "novel microorganism" hereinafter) is one type of *Bacillus subtilis*, the *Bacillus subtilis* showing a peak at at least one mass-to-charge ratio (m/z) selected from the group consisting of a range of 3045.5±0.58 (m/z), a range of 6942.5±0.58 (m/z), and a range of 9140.5±0.58 (m/z) in mass analysis by means of matrix-assisted laser desorption ionization time-of-flight mass spectrometry (MALDI-TOF MS).

### Advantageous Effects of Invention

With the present invention, it is possible to provide a novel antimicrobial agent that exhibits an antimicrobial activity against fungi and the like that infect rice plants, for example, a pesticide, a method for preventing a plant disease caused by microorganisms, and a novel *Bacillus subtilis.*

### Brief Description of Drawings

[FIG. 1] FIG. 1 shows photographs of agar media after culture in Example 1.
[FIG. 2] FIG. 2 is a graph showing the result of mass analysis in Example 1.
[FIG. 3] FIG. 3 is a photograph showing the result of electrophoresis of fractions in Example 2.
[FIG. 4] FIG. 4 is a photograph showing the antimicrobial activity of the fractions in Example 2.
[FIG. 5] FIG. 5 is a photograph showing antimicrobial activity of regions in Example 2.
[FIG. 6] FIG. 6 is a graph showing the analysis results of HPLC analyses in Example 2.
[FIG. 7] FIG. 7 shows graphs showing the analysis results of electrospray ionization liquid chromatography mass spectrometry in Example 2.

### Description of Embodiments

For example, in the antimicrobial agent of the present invention, at least one of the *Bacillus subtilis* and the culture of the *Bacillus subtilis* is an antimicrobial substance exhibiting antimicrobial activity in at least one of the *Bacillus subtilis* and the culture of the *Bacillus subtilis*,

the antimicrobial substance exhibits a molecular weight of 15 KDa or less in polyacrylamide gel electrophoresis, and

fragmentation ions of the antimicrobial substance exhibit masses within a range of 1460±1 and a range of 1474±1 in mass analysis by means of electrospray ionization mass spectrometry.

For example, in the antimicrobial agent of the present invention, the antimicrobial agent is an antimicrobial agent against a fungus. For example, the fungus is at least one fungus selected from the group consisting of fungi belonging to the genus *Pyricularia* (*Pyricularia* sp.), fungi belonging to the genus *Rhizoctonia* (*Rhizoctonia* sp.), fungi belonging to the genus *Trichoderma* (*Trichoderma* sp.), fungi belonging to the genus *Fusarium* (*Fusarium* sp.), and fungi belonging to the genus *Pythium* (*Pythium* sp.).

For example, in the prevention method of the present invention, the plant is a rice plant. For example, a seedling of a rice plant or a seed of a rice plant (also referred to as "rice seed" hereinafter) is used as the rice plant.

### Antimicrobial agent

As described above, the antimicrobial agent of the present invention is characterized by including at least one of *Bacillus subtilis* and a culture of *Bacillus subtilis*, the *Bacillus subtilis* showing a peak at at least one mass-to-charge ratio (m/z) selected from the group consisting of a range of 3045.5±0.58 (m/z), a range of 6942.5±0.58 (m/z), and a range of 9140.5±0.58 (m/z) in mass analysis by means of matrix-assisted laser desorption ionization time-of-flight mass spectrometry (MALDI-TOF MS). The antimicrobial agent of the present invention is characterized by including at least one of the *Bacillus subtilis* and the culture of *Bacillus subtilis* and showing the above-described peak, and there is no particular limitation on configurations and conditions other than this.

As a result of having conducted diligent research, the inventors of the present invention found that novel *Bacillus subtilis* (novel microorganism) showing the above-described peak in mass analysis by means of MALDI-TOF MS produces an antimicrobial substance that exhibits antimicrobial activity against microorganisms such as fungi and bacteria. The fragmentation ions of the antimicrobial substance show peaks at mass-to-charge ratios that are different from those at which antimicrobial substances such as iturin, surfactin, and fengycin produced by known *Bacillus subtilis* show peaks in mass analysis by means of liquid chromatography mass spectrometry including electrospray ionization, for example, and therefore, it is inferred that the antimicrobial substance is a novel antimicrobial substance. Therefore, with the present invention, it is possible to provide a novel antimicrobial substance exhibiting antimicrobial activity against microorganisms such as fungi and bacteria that infect rice seedlings and rice seeds, a novel antimicrobial agent including a novel microorganism including the antimicrobial substance, a culture of the novel microorganism or the like.

In the present invention, the antimicrobial activity may be bacteriostatic activity or bactericidal activity. As described later, when the antimicrobial agent is used for fungi, the antimicrobial activity may be activity for inhibiting or suppressing the growth of hyphae of the fungi, inhibiting or suppressing the formation of conidia of the fungi, or inhibiting or suppressing the formation of mycelia of the fungi. When the antimicrobial agent is used for bacteria, the antimicrobial activity may be activity for inhibiting or suppressing the growth of the bacteria, or sterilization activity.

In the present invention, the above-mentioned *Bacillus subtilis* shows at least one peak at at least one mass-to-charge ratio (m/z) selected from the group consisting of a range of 3045.5±0.58 (m/z), a range of 6942.5±0.58 (m/z), and a range of 9140.5±0.58 (m/z), and preferably the group consisting of 3046, 6943, and 9141 (m/z) or the group consisting of 3045, 6942, and 9140 (m/z), in the above-mentioned mass analysis by means of MALDI-TOF MS. The above-mentioned *Bacillus subtilis* may show one peak or a plurality of peaks out of the above-mentioned peaks, and it is preferable that the *Bacillus subtilis* shows all the peaks. There is no particular limitation on a method of culturing the *Bacillus subtilis* subjected to the above-mentioned MALDI-TOF MS and conditions thereof, and a culturing method and culturing conditions of Examples as described later can be used, for example.

An example of a mass spectrometer to be used in the above-mentioned mass analysis by means of MALDI-TOF MS is an AXIMA ® Confidence MALDI-TOF mass spectrometer (manufactured by Shimadzu Corporation), and examples of the conditions of the mass analysis are the conditions of mass analysis described below.

Conditions of mass analysis
Acceleration voltage: 20 kV (linear mode)
Ion source laser: nitrogen laser with wavelength of 337 nm
Pulse iteration value: 3 nsec
Laser beam radiation rate: 50 Hz or less
Degree of vacuum inside chamber: 1.0E-3 Pa or lower

There is no particular limitation on the culture of the above-mentioned novel microorganism, and examples thereof include cells of the novel microorganism, culture supernatant of the novel microorganism, and a cell extract of the novel microorganism. The antimicrobial agent of the present invention may further include a culture of a microorganism other than the novel microorganism. There is no particular limitation on the culture of a microorganism other than the novel microorganism, and examples thereof include cells of another microorganism, culture supernatant of another microorganism, and a cell extract of another microorganism.

The above-mentioned culture may also be a product obtained by processing the cells, a product obtained by processing the culture supernatant, a product obtained by processing the cell extract, or the like. There is no particular limitation on the processed product, and examples thereof include products obtained by concentrating the culture, drying the culture, lyophilizing the culture, processing the culture with a solvent, processing the culture with a surfactant, processing the culture with an enzyme, fractionating proteins in the culture, processing the culture with ultrasonic wave, grinding the culture, and purifying the above-mentioned antimicrobial substance. The culture may also be a mixture of the cells, the culture supernatant, the cell extract, the product obtained by processing the cells, the product obtained by processing the culture supernatant, the product obtained by processing the cell extract, and the like, for example. There is no particular limitation on the mixture, and the mixture can be obtained by mixing them in any combination at any ratio. There is no particular limitation on the combination, and an example thereof is a mixture of the cells and the culture supernatant.

It is preferable that, in the antimicrobial agent of the present invention, at least one of the above-mentioned *Bacillus subtilis* and a culture of the *Bacillus subtilis* includes an antimicrobial substance exhibiting antimicrobial activity, for example. The antimicrobial substance exhibits a molecular weight of 15 KDa or less in polyacrylamide gel electrophoresis (SDS-PAGE), while the fragmentation ions of the antimicrobial substance exhibit masses within a range of and a range of 1474±1, or masses of 1460 and 1474 in mass analysis by means of electrospray ionization analysis. The antimicrobial substance exhibits antimicrobial activity, and therefore, in the antimicrobial agent of the present invention, at least one of the *Bacillus subtilis* and the culture of the *Bacillus subtilis* may be an antimicrobial substance exhibiting antimicrobial activity in at least one of the *Bacillus subtilis* and the culture of the *Bacillus subtilis.*

An example of an electrophoresis apparatus to be used for the above-mentioned SDS-PAGE is a Mini-PROTEAN Tetra Cell System (manufactured by Bio-Rad Laboratories, Inc.), and examples of the conditions of the SDS-PAGE are the conditions of electrophoresis described below.

Conditions of electrophoresis
(1) Separating gel: 20% acrylamide
(2) Stacking gel: 4.5% acrylamide
(3) Gel size: mini gel (size (W × L): 8.3 cm × 7.3 cm)
(4) Gel thickness: 1 mm
(5) Electrophoresis buffer: 25 mmol/L Tris, 200 mmol/L glycine, and 0.1% (w/v) sodium dodecyl sulfate

Examples of an analytical instrument and a mass spectrometer to be used in the above-mentioned electrospray ionization mass spectrometry are respectively a 1260 Infinity HPLC system (manufactured by Agilent Technologies) and an Agilent G6530B Accurate-Mass Q TOF (manufactured by Agilent Technologies), and examples of the conditions of the mass analysis are the conditions of electrospray ionization liquid chromatography mass spectrometry described below.

Conditions of electrospray ionization liquid chromatography mass spectrometry
(1) Column: Monobis ODS column (manufactured by Kyoto Monotech)
(2) Mobile phase: Solvent A: 0.2% aqueous solution of acetic acid
   B: 0.2% acetic acid-methanol
   Gradient conditions: Time (solvent ratio (A:B))
   0 minutes: (A:B=98:2)
   13 minutes: (A:B=2:98)

The ratio of solvent A is reduced and the ratio of solvent B is increased in a linear manner between 0 minutes to 13 minutes such that the above solvent ratio is achieved, and then the ratio between solvents A and B is kept for 6 minutes.
(3) Flow rate: 0.6 mL/min
(4) Mode: MS Q-TOF, Dual AJS ESI ionization, positive ion mode

The antimicrobial agent of the present invention may further include other components such as additives. There is no particular limitation on the additives, and an example thereof is a stabilizer. There is no particular limitation on a method for manufacturing the antimicrobial agent. The method can be determined as appropriate depending on the dosage form of the antimicrobial agent, which will be described later, for example, and a commonly used drug-formulation technique or the like can be used, for example.

The antimicrobial agent of the present invention can be used for various microorganisms such as fungi and bacteria. Examples of the fungi include fungi belonging to the genus *Pyricularia* (*Pyricularia* sp.) such as *Pyricularia grisea* and *Pyricularia oryzae*, fungi belonging to the genus *Rhizoctonia* (*Rhizoctonia* sp.) such as *Rhizoctonia solani*, fungi belonging to the genus *Trichoderma* (*Trichoderma* sp.) such as *Trichoderma viride*, fungi belonging to the genus *Fusarium* (*Fusarium* sp.) such as *Fusarium fujikuroi* and *Fusarium moniliforme*, and fungi belonging to the genus *Pythium* (*Pythium* sp.) Examples of the bacteria include bacteria belonging to the genus *Burkholderia* (*Burkholderia* sp.) such as *Burkholderia gladioli* and *Burkholderia glumae,* and bacteria belonging to the genus *Pseudomonas* (*Pseudomonas* sp.) such as *Pseudomonas avenae and Pseudomonas plantarii.*

There is no particular limitation on a collection source of the above-mentioned novel microorganism, and examples thereof include a fertilizer, soil, sea water, river water, lake water, swamp water, and waste water. The fertilizer is preferable. Examples of the soil include soil, sand, and mud taken from dry land, the bottom of the sea, the bottom of a river, the bottom of a lake, and the bottom of a swamp.

There is no particular limitation on a method for isolating the above-mentioned novel microorganism, and a conventionally known collecting method and culturing method can be used, for example. The method for isolating the novel microorganism can be performed with reference to an isolating method of Examples, which will be described later. When a fertilizer is used as the collection source, the novel microorganism can be isolated from colonies obtained by suspending a collected fertilizer in a buffer or the like, centrifuging this suspension, and then culturing the obtained supernatant on an agar medium or the like, for example. When lake water is used as the collection source, the novel microorganism can be isolated from colonies obtained by filtering collected lake water with a filter or the like, and culturing this filtrate on an agar medium or the like, for example. When mud is used as the collection source, the novel microorganism can be isolated from colonies obtained by suspending collected mud in a buffer or the like, centrifuging this suspension, and then culturing the obtained supernatant on an agar medium or the like, for example. The isolated novel microorganism may be further cultured in a liquid medium, for example.

There is no particular limitation on a medium used to culture the novel microorganism, and examples of the medium include an E5 liquid medium or an E5 solid medium, a cornmeal liquid medium or a cornmeal solid medium (manufactured by Fluka, Catalog No.: 42347-500G-F), an LB liquid medium or an LB solid medium, and a potato dextrose liquid medium or a potato dextrose solid medium, which will be described later.

There is no particular limitation on a temperature at which the above-described novel microorganism is cultured, and examples thereof include a temperature of 25 to 55°C, a temperature of 25 to 35°C, and a temperature of 45 to 55°C.

There is no particular limitation on a culture pH during the culture, and examples thereof include a pH within a range of pH 4.5 to 7.5, a pH within a range of pH 6.6 to 7.5, or a pH within a range of pH 4.5 to 5.5.

The culture may be performed in an aerobic condition or in an anaerobic condition. There is no particular limitation on the aerobic condition and the anaerobic condition, and these conditions can be set using a conventionally known method. There is no particular limitation on a light condition during the culture, and the culture may be performed in a dark condition or in a lighted, for example.

There is no particular limitation on a period of time for the culture, and the culture may be performed until the growth of the novel microorganism reaches a stationary phase. When a culture condition is such that the growth of the novel microorganism reaches a stationary phase in about 72 hours, the culture may be performed for 72 hours, for example.

For example, during the culture of the novel microorganism, only the novel microorganism may be cultured, or the novel microorganism and other microorganisms may be cultured together in a mixed manner. There is no particular limitation on the other microorganisms.

### Pesticide

As described above, the pesticide of the present invention is characterized by including the antimicrobial agent of the present invention. The pesticide of the present invention is characterized by including the antimicrobial agent of the present invention, and there is no particular limitation on configurations and conditions other than this. With the pesticide of the present invention, a plant disease caused by microorganisms can be prevented, for example. The description of the antimicrobial agent of the present invention can be applied to the pesticide of the present invention, for example.

There is no particular limitation on the dosage form of the pesticide of the present invention, and examples thereof include solid medicines such as a powder medicine, a fine granular medicine and a granular medicine, liquid medicines, and emulsions. The pesticide of the present invention may also include a known pesticide, for example. The known pesticide may be a biological pesticide or a chemical pesticide. The biological pesticide contains microorganisms (e.g., fungi and bacteria) that have activity such as antimicrobial activity, insecticidal activity, bactericidal activity, weeding activity, plant growth controlling activity, or insect repelling activity. Examples of the chemical pesticide include an antimicrobial substance, an insecticidal substance, a bactericidal substance, a weeding substance, a plant growth controlling substance, and an insect repelling substance.

There is no particular limitation on the subjects to be treated with the pesticide, and examples thereof include plants, soil in which the plants are cultivated, and a medium such as water. Examples of the plants include gramineous plants such as a rice plant. When the pesticide is used for the plants, there is no particular limitation on the portions of the plants with which the pesticide is brought into contact, and the pesticide may be brought into contact with the entireties of individual plants or portions of individual plants. Examples of the portions of the individual plants include organs, tissues, cells and propagules, and the pesticide may be brought into contact with any of the portions. Examples of the organs include petals, corollas, flowers, leaves, seeds, fruits, stems and roots. Specific examples of the targets include seeds of a rice plant (rice seeds) and seedlings of a rice plant. There is no particular limitation on the growth state of the plants when the pesticide is used, and the pesticide may be used for seeds, seedlings, or grown plants.

There is no particular limitation on the usage amount and usage frequency of the above-mentioned pesticide, and the usage amount and usage frequency can be determined as appropriate depending on the targets.

The pesticide of the present invention can be used according to a common usage method, for example. Examples of the usage method include a method of sprinkling the pesticide directly by hand, and methods of applying granules using a granule applicator such as a knapsack granule applicator, a pipe granule applicator, an aerial granule applicator, a power granule applicator, a granule applicator for a seedling box, a granule applicator with a multi-hole hose, or a granule applicator mounted in a rice-planting machine. When the pesticide of the present invention is in the form of a liquid medicine, the pesticide can be sprinkled using a sprinkling apparatus such as a knapsack sprinkling apparatus, a power sprinkling apparatus, a sprinkler, a sprinkling apparatus mounted on a tractor or the like, and a sprinkling apparatus with a multi-hole hose.

There is no particular limitation on places in which the pesticide of the present invention is used, and the pesticide of the present invention can be used in agricultural land such as a paddy field, a dry rice field, a seedling box, a farmland, an orchard, a mulberry field, a greenhouse, or bare ground, or the like.

A case where the pesticide of the present invention is used for a rice plant will be described by use of examples. When the pesticide of the present invention is used for a rice plant, the pesticide of the present invention can be used during seeding, seedling raising, rice planting, or the like. The pesticide of the present invention may be used in a non-diluted state or in a diluted state, for example. When used in the paddy field, for example, the pesticide of the present invention in a granular form or in a liquid form can be applied or sprinkled using the above-described granule applicator, sprinkling apparatus, sprinkler, or the like. When used to disinfect rice seeds of the rice plant, the pesticide of the present invention can be used as a coating agent for the rice seeds of the rice plant, for example. Furthermore, for example, the pesticide of the present invention may be used in a state of being dissolved in water when the rice seeds of the rice plant are soaked in water, or used in a state of being dissolved in warm water when the rice seeds of the rice plant are disinfected, or be sprayed on water when the rice seeds of the rice plant are forced to sprout.

There is no particular limitation on the usage amount and usage frequency of the pesticide of the present invention when the pesticide is used in the paddy field, and the usage amount and usage frequency can be determined as appropriate depending on the size of the paddy field and the usage method.

### Method for preventing plant disease caused by microorganisms

As described above, the method for preventing a plant disease caused by microorganisms of the present invention includes a contacting step of bringing the antimicrobial agent of the present invention into contact with a plant. The prevention method of the present invention is characterized by using the antimicrobial agent of the present invention, and there is no particular limitation on steps and conditions other than this. With the prevention method of the present invention, a plant disease caused by microorganisms such as the fungi and the bacteria can be prevented in the plant such as a seedling or a grown plant, for example. The descriptions of the antimicrobial agent, pesticide, and the like of the present invention can be applied to the prevention method of the present invention, for example.

In the present invention, "preventing a plant disease caused by the microorganisms" means an ability to inhibit or suppress the development or progress of a disease caused by infection by the microorganisms, and specifically, any of allowing no diseases to be developed, stopping the progress of a disease that has been developed, suppressing (also referred to as "inhibiting") the progress of a disease that has been developed, and the like is possible.

In the above-mentioned contacting step, the antimicrobial agent of the present invention is brought into contact with a plant. In the contacting step, the above-mentioned pesticide of the present invention may be used as the antimicrobial agent of the present invention and be brought into contact with the plant. The description of the pesticide of the present invention can be applied to a method for bringing the antimicrobial agent of the present invention into contact with the plant, for example.

### Novel Bacillus subtilis

As described above, the novel *Bacillus subtilis* of the present invention is one type of *Bacillus subtilis*, and is characterized by showing a peak at at least one mass-to-charge ratio (m/z) selected from the group consisting of a range of 3045.5±0.58 (m/z), a range of 6942.5±0.58 (m/z), and a range of 9140.5±0.58 (m/z) in mass analysis by means of matrix-assisted laser desorption ionization time-of-flight mass spectrometry (MALDI-TOF MS). The novel microorganism of the present invention is one type of *Bacillus subtilis*, and is characterized in that the *Bacillus subtilis* shows the above-described peak in mass analysis by means of MALDI-TOF MS. There is no particular limitation on configurations and conditions other than this. With the novel microorganism of the present invention, the antimicrobial agent and the pesticide of the present invention can be manufactured, for example. The descriptions of the antimicrobial agent, pesticide, and prevention method of the present invention can be applied to the novel microorganism of the present invention, for example.

### Examples

Next, examples of the present invention will be described. However, the present invention is not limited to the following examples. Commercially available reagents were used based on their operating instruction manuals.

### Example 1

The novel microorganism of the present invention was isolated, and it was confirmed that the novel microorganism produced an antimicrobial substance exhibiting antimicrobial activity.

### (1) Isolation of novel microorganism

An organic fertilizer collected in Yamagata Prefecture was used as an isolation source, and the novel microorganism was isolated therefrom. An E5 medium was used as a medium for isolation. A stock medium for an E5 medium was prepared by mixing the components of the composition shown in Table 1 below, and then stored in a dark and cool place. Next, 2.4 g of the stock medium was weighed and added to 1.2 L of distilled water, and the mixture was boiled for 5 minutes. After the boil, the resultant solution was filtered to remove impurities, and then the filtrate was collected to a container. Furthermore, the collected solution was sterilized in an autoclave at 121°C for 20 minutes, and then cooled. An E5 medium was thus prepared.

**Table 1**

| Components | Weight (kg) |
|---|---|
| Crab shell powder | 1.5 |
| Sea tangle powder | 1.5 |
| Oyster shell powder | 2.3 |
| Dried bonito powder | 1.5 |
| Thin wheat noodle powder | 3 |
| Rice powder | 2.1 |
| Soy sauce lee | 1 |
| Peanut powder | 1 |
| Corn powder | 2 |
| Fish powder | 2 |
| Rice chaff | 0.8 |
| Rice bran | 1.8 |
| Silica powder | 1 |
| Rapeseed cake | 1.2 |
| Buckwheat chaff powder | 0.8 |
| Total | 23.5 |

Agar was added to 1 L of the E5 medium to give a final concentration of 1.5% w/v. The resultant mixture was dissolved and sterilized in an autoclave at 121°C for 20 minutes, and then poured into a petri dish at an appropriate temperature (in a liquid state), and thereafter was cooled and solidified. An E5 agar plate medium (E5 agar medium) was thus prepared.

An appropriate amount of the E5 medium was poured into a flask, and then 250 mg of the above-mentioned isolation source was placed in the flask and cultured at a rotation rate of 120 rpm at 30 to 48 °C for 24 to 144 hours. A portion of the resultant culture solution was spread on the E5 agar medium and cultured until the formation of a colony was observed. Bacteria were separated from each colony that had been formed with a sterilized toothpick, distinguished by the shape, color, odor, and the like of the colony, and spread on another E5 agar medium. Enrichment culture was performed by repeating the similar operation multiple times, and 72 strains of microorganisms that had different features were thus obtained. These strains were named YAE strains. The above-mentioned novel microorganism (YAE51 strain) was the 51st *Bacillus subtilis* obtained by the above-mentioned isolation method.

### (2) Confirmation of antimicrobial activity

After 1.25 g of Bacto (registered trademark) yeast extract (manufactured by Becton Dickinson, Catalog No.: 212750) was added to 1 L of the E5 medium, agar was added thereto to give a final concentration of 1.5% w/v. The resultant mixture was dissolved and sterilized in an autoclave at 121°C for 20 minutes, and then poured into a petri dish at an appropriate temperature (in a liquid state), and thereafter was cooled and solidified. An E5 yeast agar plate medium (E5 yeast agar medium) was thus prepared.

An agar block with a size of 0.5 cm × 0.5 cm obtained from an E5 yeast agar medium on which a fungal strain had been separately cultured was inoculated at the center of another E5 yeast agar medium. *Fusarium fujikuroi* MAFF235949 (a rice "Bakanae" fungus, obtained from the NARO Genebank), *Pyricularia grisea* MAFF101518 (a rice blast fungus, obtained from the NARO Genebank), and *Rhizoctonia solani* MAFF305229 (a rice sheath blight fungus, obtained from the NARO Genebank) were used as the fungal strains.

Furthermore, after the inoculation, the novel microorganism was inoculated around the fungal strain with a sterilized toothpick. The E5 yeast agar medium was cultured at 30°C for a predetermined number of days (the rice "Bakanae" fungus: 5 days, the rice blast fungus: 3 days, and the rice sheath blight fungus: 3 days). After the culture, the diameter (R) of an inhibition ring formed between the novel microorganism and each fungal strain was evaluated based on the evaluation standards of antimicrobial activity described below. It should be noted that the average value of the long diameter and the short diameter was used as the diameter of the inhibition ring. In addition, the evaluation was performed in the same manner as described above, except that a corn meal agar medium (manufactured by Fluka, Catalog No.: 42347-500G-F) was used instead of the E5 yeast agar medium, and *Trichoderma viride* NBRC30546 (rice dieback, obtained from the National Institute of Technology and Evaluation) was used as the fungal strain and cultured for a predetermined number of days (rice dieback: 3 days).

### Evaluation standards of antimicrobial activity

+++: 5≤R (mm)
++ : 1<R<5 (mm)
+ : 0<R≤1 (mm)
- : 0=R (mm)

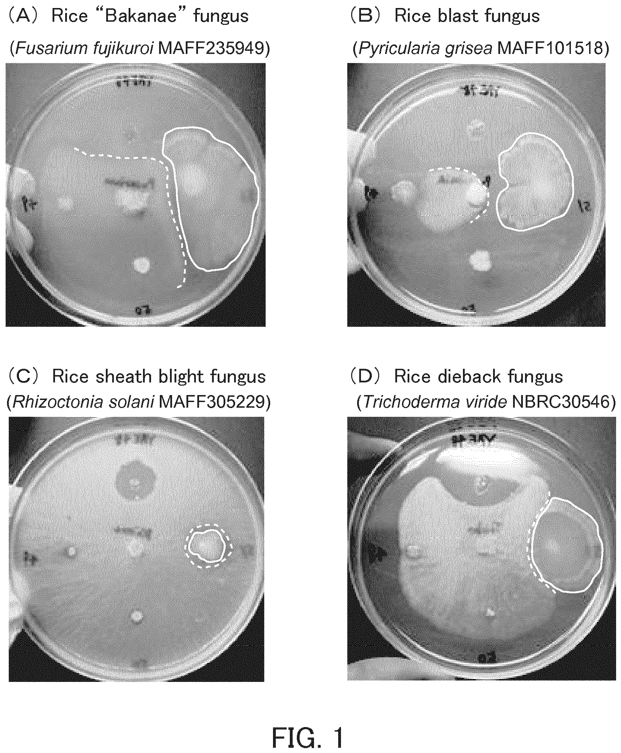

FIG. 1 shows the results. FIG. 1 shows photographs of agar media after the culture. FIG. 1(A) shows the result from the rice "Bakanae" fungus, FIG. 1(B) shows the result from the rice blast fungus, FIG. 1(C) shows the rice sheath blight fungus, and FIG. 1(D) shows the result from the rice dieback. In each of the diagrams, a solid line indicates a region in which the novel microorganism has grown, a broken line indicates a region in which the fungal strain has grown, and a region between the solid line and the broken line indicates the inhibition ring. As shown in FIG. 1, the novel microorganism exhibited antimicrobial activity against all of the fungal strains.

Next, Table 2 below shows the diameters (R) of the inhibition rings and the evaluations of antimicrobial activity. As shown in Table 2 below, the novel microorganism exhibited antimicrobial activity against all of the fungal strains. In particular, the novel microorganism exhibited high antimicrobial activity against the rice blast fungus and the rice sheath blight fungus.

**Table 2**

| | Rice "Bakanae" fungus | Rice blast fungus | Rice sheath blight fungus | Rice dieback fungus |
|---|---|---|---|---|
| Diameter (R) (mm) | 1.5 | 5 | 2.5 | 0.9 |
| Antimicrobial activity | ++ | +++ | ++ | + |

### (3) Identification of novel microorganism

To a vial in which 5 mg of matrix 4-CHCA powder (manufactured by Shimadzu GLC Ltd., Catalog No.: 529-CHCA), which had been stored in a refrigerator, had been placed, 67 µL of 100% acetonitrile, 67 µL of 100% ethanol, and 67 µL of 10% TFA/H₂O were added, and then the mixture was suspended sufficiently. A matrix reagent in which the final concentration of the matrix was 25 mg/ml was thus prepared.

A sterilized toothpick was used to separate bacteria from a colony of the novel microorganism in the growth phase on the E5 yeast agar medium and apply the bacteria onto a well of a sample plate (manufactured by Shimadzu GLC Ltd.). Next, 1 µL of the matrix reagent was added to the well, and then the well was dried at room temperature (about 25°C) for 30 minutes to 1 hour until the moisture in the matrix reagent was completely lost.

The novel microorganism was identified by using a mass spectrometer (AXIMA (registered trademark) Confidence MALDI-TOF mass spectrometer, manufactured by Shimadzu Corporation) to perform mass analysis in measurement conditions described below. The attached software (LAUNCHPAD, manufactured by Shimadzu Corporation) was used to control the mass spectrometer and collect mass data. It should be noted that a mass range that can be measured in mass analysis in the measurement conditions described below is a range of 1 Da to 500 kDa.

### Conditions of mass analysis

Acceleration voltage: 20 kV (linear mode)
Ion source laser: nitrogen laser with wavelength of 337 nm
Pulse iteration value: 3 nsec
Laser beam radiation rate: 50 Hz or less
Degree of vacuum inside chamber: 1.0E-3 Pa or lower

The novel microorganism strain was identified using software (SARAMIS (registered trademark) Premium, manufactured by bioMerieux) based on the obtained mass data and the mass data of proteins derived from six types of standard bacterial strains registered by bioMerieux. The identification was performed based on the following reference. Similar identification was repeated three more times. The reference is as follows: Lohmann C et. al.,"Comparison between the Biflex III-Biotyper and the Axima-SARAMIS Systems for Yeast Identification by Matrix-Assisted Laser Desorption Ionization-Time of Flight Mass Spectrometry", J Clin Microbiol., 2013, vol.51, No. 4, pages.1231-1236.

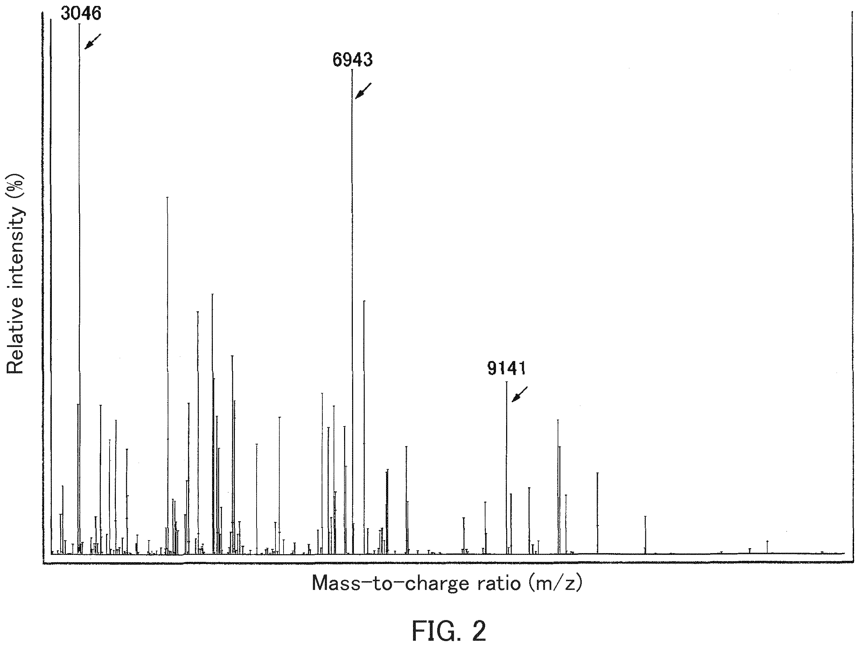

FIG. 2 shows the results. FIG. 2 is a graph showing the result of mass analysis. FIG. 2 shows the results from the novel microorganism and the standard bacterial strains. The numbers in the diagram indicate peak values (m/z), and the arrows indicate peaks that are present in the result from the novel microorganism but not present in the result from the standard bacterial strains. In FIG. 2, the horizontal axis indicates the mass-to-charge ratio (m/z), and the vertical axis indicates the relative intensity (%). As shown in FIG. 2, a large number of peaks match between the mass data of the novel microorganism and the mass data of the proteins of the standard bacterial strains. The peaks at 3046, 6943, and 9141 (m/z) are present in the mass data of the novel microorganism, whereas these peaks are not present in the mass data of the proteins of the standard bacterial strains. As a result of determining the average value of this result and the results obtained by repeating the identification three more times, it was found that the novel microorganism shows peaks within a range of 3045.5±0.58 (m/z), a range of 6942.5±0.58 (m/z), and a range of 9140.5±0.58 (m/z).

Next, Table 3 below shows the peak matching rate between the mass data of the novel microorganism and the mass data of the six types of standard bacterial strains. As shown in Table 3, the peak matching rate between the novel microorganism and the bacterial strain belonging to *Bacillus subtilis* was significantly high. It was found from these results that the novel microorganism was a novel microorganism belonging to *Bacillus subtilis.* It should be noted that it was found that, in the mass data of the novel microorganism, the peaks within a range of 3045.5±0.58 (m/z), a range of 6942.5±0.58 (m/z), and a range of 9140.5±0.58 (m/z) were different from the peaks in the mass data of these bacterial strains, and the novel microorganism of the present invention could be identified by these peaks.

**Table 3**

| Standard bacterial strains | Family | Genus | Species | Matching rate (%) |
|---|---|---|---|---|
| Bacillus subtilis 2 | Bacillales | Bacillus | subtilis | 99.90 |
| Bacillus subtilis 4 | Bacillales | Bacillus | subtilis | 99.90 |
| Bacillus subtilis 0 DSM10 | Bacillales | Bacillus | subtilis | 99.90 |
| Bacillus subtilis 3 | Bacillales | Bacillus | subtilis | 96.00 |
| Bacillus atrophaeus/subtilis | Bacillales | Bacillus | atrophaeus/subtilis | 88.50 |
| Bacillus sp. | Bacillales | Bacillus | sp. | 85.20 |

### Example 2

An antimicrobial substance exhibiting the antimicrobial activity was purified from the novel microorganism of the present invention and then analyzed.

### (1) Purification using gel filtration column

In a 1-L baffled Erlenmeyer flask, 200 mL of an LB medium was placed and sterilized in an autoclave at 121°C for 20 minutes. The LB medium had a composition containing 1% Bacto (registered trademark) Trypton (manufactured by Becton Dickinson, Catalog No.: 211705), 0.5% Bacto (registered trademark) yeast extract, and 1% NaCl (manufactured by Wako Pure Chemical Industries, Ltd.: Catalog No.: 191-01665). The novel microorganism was inoculated in the LB medium and cultured at a shaking rotation rate of 120 rpm at 30°C for 48 hours.

After the culture, the culture solution was centrifuged at 8000xg for 20 minutes. Next, after the bacterial cells were removed by collecting the supernatant, ammonium sulfate (manufactured by Wako Pure Chemical Industries, Ltd.: Catalog No.: 013-03433) was added to the supernatant to give a final concentration of 30%. Thereafter, the resultant solution was left to stand at 4°C for 1 hour and then centrifuged at 10000×g for 20 minutes, and a precipitated substance (precipitate) was thus collected.

The collected precipitate was dissolved in 2 mL of 20 mmol/L Tris-HCl (pH 8.0), and a crude sample was thus prepared. The crude sample was placed in a cellulose tube 18/32 (manufactured by EIDIA Co., Ltd., Catalog No.: UC18-32-100) and desalted (also referred to as "dialyzed" hereinafter) in 1 L of a dialysate, and a crude antimicrobial substance solution was thus obtained. As the dialysate, 20 mmol/L Tris-HCl (pH 8.0) buffer was used. The dialysis was performed at 4°C overnight. The dialysate was replaced once during the dialysis.

Next, the crude antimicrobial substance solution was subjected to gel filtration performed in the purification conditions of gel filtration described below, and 912th, 21st, 24th, 27th and 30th fractions were obtained. It should be noted that, in the gel filtration, 250 mL of a column equilibration buffer was poured into the column, and then 1 mL of the crude antimicrobial substance solution was poured thereinto, followed by 180 mL of a mobile phase. The fractions were collected while the mobile phase flowed.

### Purification conditions of gel filtration

(1) Apparatus: Low-pressure normal-phase chromatograph (AKTA prime plus (manufactured by GE Healthcare Bioscience))
(2) Column: HiPrep 16/60 Sephacryl S-200HR (manufactured by GE Healthcare Bioscience, Catalog No.: 17116601)
(3) Column equilibration buffer and mobile phase: 20 mmol/L Tris-HCl, 150 mmol/L NaCl (pH 8.0)
(4) Flow rate: 0.5 mL/min
(5) Collection amount: 5 mL/fraction

Next, each of the fractions was concentrated to 20 times the initial concentration (one twentieth of the initial volume) by ultrafiltration using Amicon Ultra-0.5 (manufactured by Merck Millipore, Catalog No.: UFC501096), and a fraction sample was thus prepared. Each of the crude antimicrobial substance solution and the fraction samples was mixed with an equal amount of ×2 SDS sample buffer and boiled at 100°C for 3 minutes. After boiling, the crude antimicrobial substance solution and the fraction samples were subjected to SDS-PAGE performed in the conditions of electrophoresis described below using an electrophoresis apparatus (mini-PROTEAN Tetra Cell System, manufactured by Bio-Rad Laboratories, Inc.). It should be noted that the electric current value was set to 30 mA, and the electrophoresis was performed for 40 minutes.

### Conditions of electrophoresis

(1) Separating gel: 20% acrylamide
(2) Stacking gel: 4.5% acrylamide
(3) Gel size: mini gel (size (W × L): 8.3 cm × 7.3 cm)
(4) Gel thickness: 1 mm
(5) Electrophoresis buffer: 25 mmol/L Tris (manufactured by Wako Pure Chemical Industries, Ltd., Catalog No.: 207-06275), 200 mmol/L glycine (manufactured by Wako Pure Chemical Industries, Ltd., Catalog No.: G8898-1KG), and 0.1% (w/v) sodium dodecyl sulfate (manufactured by Nacalai Tesque Inc., Catalog No.: 31607-65)

After the electrophoresis, the acrylamide gel was treated with a fixative and then stained with a staining solution containing Coomassie brilliant blue (CBB) (manufactured by Wako Pure Chemical Industries, Ltd., Catalog No.: 031-17922) for 30 minutes. The fixative had a composition containing 50% ethanol and 10% acetic acid. After the staining, the acrylamide gel was destained by removing the CBB dye using a destaining solution. The destaining solution had a composition containing 25% methanol and 7.5% acetic acid. The destaining treatment was performed for 3 hours, and the destaining solution was replaced several times.

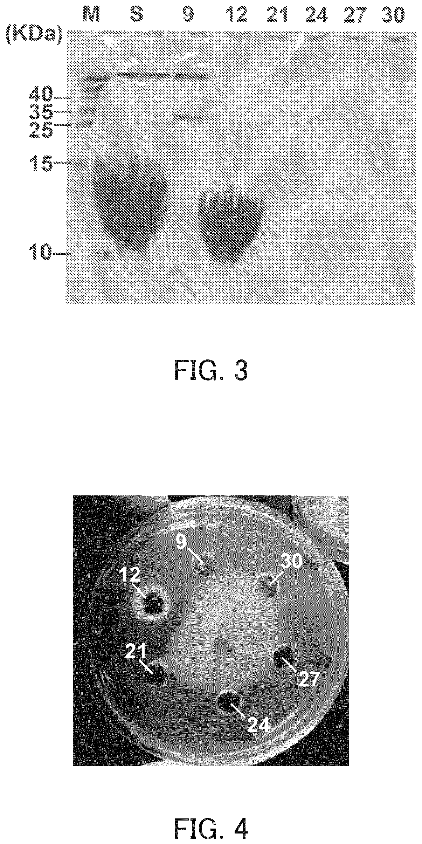

FIG. 3 shows the results. FIG. 3 is a photograph showing the results of electrophoresis of the fractions. In FIG. 3, the molecular weights are shown on the left side of the photograph. A molecular weight marker, the crude antimicrobial substance solution, and the 9th, 12th, 21st, 24th, 27th and 30th fractions were respectively applied to the lanes in the order from left to right. As shown in FIG. 3, the antimicrobial substance contained in the crude antimicrobial substance solution was purified and contained in the 12th fraction, and exhibited a molecular weight of 15 kDa or less in SDS-PAGE.

Next, the antimicrobial activity of each of the fractions was examined using well diffusion assay. First, a medium was prepared using Difco (registered trademark) Potato Dextrose Agar (manufactured by Becton Dickinson, Catalog No.: 21340) based on the attached operating instruction manual. Specifically, 39 g of Difco (registered trademark) Potato Dextrose Agar was weighed and placed in a container containing 1 L of distilled water, and then dissolved. The container and the medium were sterilized in an autoclave at 121°C for 20 minutes, and then the medium was poured into a plastic petri dish at an appropriate temperature (in a liquid state), and thereafter was cooled and solidified. A Potato Dextrose Agar plate medium was thus prepared.

Next, *Rhizoctonia solani* MAFF305229 (rice sheath blight fungus) was used as the fungal strain and cultured in the same manner as in Example 1 (2) described above, except that 100-µL aliquots of the 9th, 12th, 21st, 24th, 27th and 30th fractions were used instead of the novel microorganism, and were respectively placed in six holes with a diameter of about 0.8 mm formed around the fungal strain.

FIG. 4 shows the results. FIG. 4 is a photograph showing the antimicrobial activity of the fractions. As shown in FIG. 4, the inhibition ring formed by the 12th fraction was significantly large, and it was thus found that an antimicrobial substance was contained in the 12th fraction. Accordingly, it was found that the antimicrobial substance exhibited a molecular weight of 15 KDa or less in SDS-PAGE.

The 12th fraction was subjected to SDS-PAGE using a 20% acrylamide gel. The 12th fraction was applied to a plurality of lanes. After the electrophoresis, one lane was stained with CBB. Then, the lane stained with CBB was used as a standard, and the lanes that had not been stained with CBB were divided into three regions, namely a region (second region) in which a band is observed, a region (first region) that corresponds to higher molecular weights than those to which the second region corresponds, and a region (third region) that corresponds to lower molecular weights than those to which the second region corresponds. The three divided regions were washed with ultrapure water.

In the next step, *Rhizoctonia solani* MAFF305229 (rice sheath blight fungus) was used as the fungal strain. The three washed regions were placed on the E5 yeast agar plate medium instead of the novel microorgansim, and then 10 mL of a sterilized E5 yeast agar solution was layered thereon to fix the gels. Next, an agar block with sides of about 5 mm × 5 mm in which *Rhizoctonia solani* MAFF305229 was growing was inoculated around the gels. After the inoculation, culture was performed at 30°C for 3 days. The antifungal reaction was evaluated by the size of an inhibition ring that indicates the inhibition of the growth of the rice sheath blight fungus.

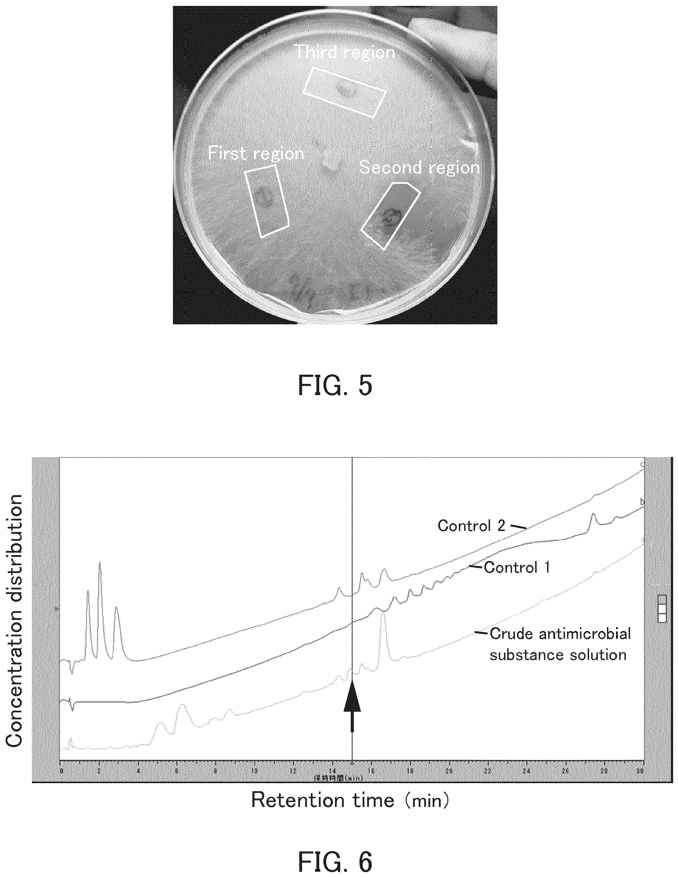

FIG. 5 shows the results. FIG. 5 is a photograph showing the antimicrobial activity of the regions. As shown in FIG. 5, the formation of hyphae was not observed in the second region. In contrast, the formation of hyphae was observed in the first and third regions. It was found from these results that the antimicrobial substance exhibited a molecular weight of 15 KDa or less in SDS-PAGE.

### (2) HPLC analysis

The crude antimicrobial substance solution was extracted with an organic solvent (1-butanol), and then the extract was evaporated to dryness. A crude sample was prepared by dissolving the dried residue in ethanol and was then subjected to HPLC analysis. The crude sample was subjected to reversed phase HPLC (high performance liquid chromatography) performed in the analysis conditions of reversed phase HPLC described below using an analytical instrument (LaChrom HPLC system, manufactured by Hitachi High-Tech Science Corporation). Control 1 was analyzed in the same manner, except that methanol was used instead of the crude sample, and control 2 was analyzed in the same manner, except that a methanol solution containing 1.0 mg/mL A-type iturin and 1.0 mg/mL surfactin was used.

### Analysis conditions of reversed phase HPLC

(1) Column: Monobis ODS column (manufactured by Kyoto Monotech, high-pressure resistant type, mesopore diameter of 11 nm, inner diameter of 2.0 mm × 50 mm)
(2) Mobile phase: solvent A: mixed solution of 0.1% formic acid (manufactured by Kanto Chemical Co., Inc., Catalog No.: 16233-00)-acetonitrile (manufactured by Kanto Chemical Co., Inc., Catalog No.: 01031-96) and 2-propanol (manufactured by Kanto Chemical Co., Inc., Catalog No.: 32435-80) (mixing ratio: formic acid-acetonitrile:2-propanol=3:7)
   B: 0.1% aqueous solution of formic acid
   Gradient conditions: Time (solvent ratio (A:B))
   0 minutes: (A:B=40:60)
   30 minutes: (A:B=100:0)

The ratio of solvent B is reduced and the ratio of solvent A is increased in a linear manner between 0 minutes to 30 minutes such that the above solvent ratio is achieved.

Flow rate 0.15 mL/min
(3) Measurement wavelength: 205 nm
(4) Column temperature: 40°C

FIG. 6 shows the results. FIG. 6 is a graph showing the analysis results of the HPLC analyses. In FIG. 6, the horizontal axis indicates the retention time, and the vertical axis indicates the concentration distribution. As shown in FIG. 6, the crude sample showed a peak that was not present in the results from the controls 1 and 2 at a position indicated by an arrow in the diagram. It was found from these results that the crude sample contained an antimicrobial substance different from known antimicrobial substances.

### (3) Electrospray ionization liquid chromatography mass spectrometry analysis

The crude sample was measured using an analytical instrument (1260 Infinity HPLC system, manufactured by Agilent Technologies) and a mass spectrometer (Agilent G6530B Accurate-Mass Q TOF, manufactured by Agilent Technologies) in the conditions of electrospray ionization liquid chromatography mass spectrometry described below. Attached software (MassHunter Workstation Software Qualitative Analysis Version B.06.00, manufactured by Agilent Technologies) was used to control the mass spectrometer and collect mass data. A control was analyzed in the same manner, except that a methanol solution containing 1.0 mg/mL A-type iturin and 1.0 mg/mL surfactin was used.

Conditions of electrospray ionization liquid chromatography mass spectrometry
(1) Column: Monobis ODS column
(2) Mobile phase: Solvent A: 0.2% aqueous solution of acetic acid (manufactured by Kanto Chemical Co., Inc., Catalog No.: 01021-00)
   B: 0.2% acetic acid-methanol (manufactured by Kanto Chemical Co., Inc., Catalog No.: 25185-76)
   Gradient conditions: Time (solvent ratio (A:B))
   0 minutes: (A:B=98:2)
   13 minutes: (A:B=2:98)

The ratio of solvent A is reduced and the ratio of solvent B is increased in a linear manner between 0 minutes to 13 minutes such that the above solvent ratio is achieved, and then the ratio between solvents A and B is kept for 6 minutes.
(3) Flow rate: 0.6m L/min
(4) Mode: MS Q-TOF, Dual AJS ESI ionization, positive ion mode

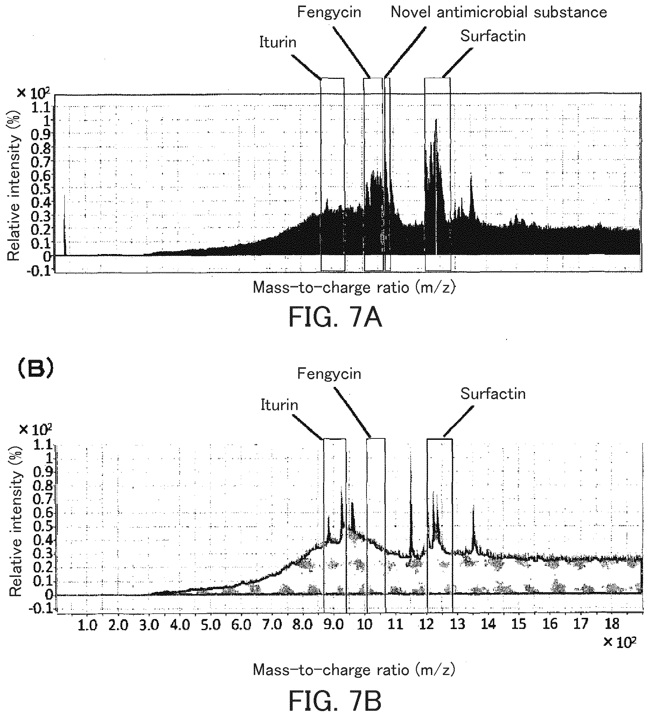

FIG. 7 shows the results. FIG. 7 shows graphs showing the analysis results of the electrospray ionization liquid chromatography mass spectrometry. FIG. 7(A) shows the result from the crude sample, and FIG. 7(B) shows the result from the control. In FIG. 7, the horizontal axis indicates the mass-to-charge ratio (m/z), and the vertical axis indicates the relative intensity (%). As shown in FIG. 7, the crude sample showed peaks at mass-to-charge ratios of 731 and 738 (m/z), which are different from the peaks indicating known antimicrobial substances, namely A-type iturin, fengycin, and surfactin, and these peaks indicated the fragmentation ions of a novel antimicrobial substance. It was found from the mass-to-charge ratios that the masses (molecular weights) of the fragmentation ions of the novel antimicrobial substance were 1460 and 1474, respectively. It was found from these results that the novel microorganism produced the novel antimicrobial substance showing peaks indicating the fragmentation ions with masses of 1460 and 1474 in mass analysis by means of electrospray ionization liquid chromatography mass spectrometry.

Although the present invention has been described with reference to the embodiments and examples, the present invention is not limited to the embodiments and examples described above. Various modifications that can be understood by a person skilled in the art can be made in the configurations and details of the present invention without departing from the scope of the present invention.

### Industrial Applicability

As described above, with the present invention, a novel antimicrobial agent including a novel antimicrobial substance exhibiting antimicrobial activity against microorganisms such as fungi and bacteria that infect rice plants, for example, a novel microorganism including the antimicrobial substance, a culture of the novel microorganism, or the like can be provided. Therefore, the present invention is very useful in fields of agriculture, medicine, environment, and the like.

## Claims

1. An antimicrobial agent comprising at least one of *Bacillus subtilis* and a culture of *Bacillus subtilis*,
wherein the *Bacillus subtilis* is a microorganism showing a peak at at least one mass-to-charge ratio (m/z) selected from the group consisting of a range of 3045.5±0.58 (m/z), a range of 6942.5±0.58 (m/z), and a range of 9140.5±0.58 (m/z) in mass analysis by means of matrix-assisted laser desorption ionization time-of-flight mass spectrometry (MALDI-TOF MS).

2. The antimicrobial agent according to claim 1,
wherein at least one of the *Bacillus subtilis* and the culture of the *Bacillus subtilis* is an antimicrobial substance exhibiting antimicrobial activity in at least one of the *Bacillus subtilis* and the culture of the *Bacillus subtilis*,
the antimicrobial substance exhibits a molecular weight of 15 KDa or less in polyacrylamide gel electrophoresis, and
fragmentation ions of the antimicrobial substance exhibit masses within a range of and a range of in mass analysis by means of electrospray ionization mass spectrometry.

3. The antimicrobial agent according to claim 1 or 2, wherein the antimicrobial agent is an antimicrobial agent against a fungus.

4. The antimicrobial agent according to claim 3, wherein the fungus is at least one fungus selected from the group consisting of fungi belonging to the genus *Pyricularia* (*Pyricularia* sp.), fungi belonging to the genus *Rhizoctonia* (*Rhizoctonia* sp.), fungi belonging to the genus *Trichoderma* (*Trichoderma* sp.), fungi belonging to the genus *Fusarium* (*Fusarium* sp.), and fungi belonging to the genus *Pythium* (*Pythium* sp.).

5. A pesticide comprising the antimicrobial agent according to any one of claims 1 to 4.

6. A method for preventing a plant disease caused by microorganisms, the method comprising a contacting step of bringing the antimicrobial agent according to any one of claims 1 to 4 into contact with a plant.

7. The prevention method according to claim 6, wherein the plant is a rice plant.

8. The prevention method according to claim 7, wherein a seedling of a rice plant or a seed of a rice plant is used as the rice plant.

9. Novel *Bacillus subtilis* that is one type of *Bacillus subtilis*
the *Bacillus subtilis* showing a peak at at least one mass-to-charge ratio (m/z) selected from the group consisting of a range of 3045.5±0.58 (m/z), a range of 6942.5±0.58 (m/z), and a range of 9140.5±0.58 (m/z) in mass analysis by means of matrix-assisted laser desorption ionization time-of-flight mass spectrometry (MALDI-TOF MS).
